(19) Europäisches Patentamt / European Patent Office / Office européen des brevets

(11) **EP 4 389 092 B1**

(12) **EUROPÄISCHE PATENTSCHRIFT**

(45) Veröffentlichungstag und Bekanntmachung des Hinweises auf die Patenterteilung:
**10.09.2025   Patentblatt 2025/37**

(21) Anmeldenummer: **23217021.7**

(22) Anmeldetag: **15.12.2023**

(51) Internationale Patentklassifikation (IPC):
**A61F 13/472** (2006.01)      **A61F 13/475** (2006.01)
**A61F 13/513** (2006.01)      **A61F 13/514** (2006.01)
**A61F 13/84** (2006.01)

(52) Gemeinsame Patentklassifikation (CPC):
**A61F 13/51496; A61F 13/47263; A61F 13/4757;
A61F 13/51394;** A61F 2013/8497

(54) **HYGIENEARTIKEL MIT ELASTIFIZIERUNG**

HYGIENE ARTICLE WITH ELASTICATION

ARTICLE D'HYGIÈNE ÉLASTIFIÉ

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität:  **20.12.2022   DE 102022134190**

(43) Veröffentlichungstag der Anmeldung:
**26.06.2024   Patentblatt 2024/26**

(73) Patentinhaber: **Paul Hartmann AG
89522 Heidenheim (DE)**

(72) Erfinder:
• **Herzog, Moritz
  73207 Plochingen (DE)**
• **Bährle, Christian
  89542 Herbrechtingen (DE)**
• **Horn, Annette
  89233 Neu-Ulm (DE)**

(74) Vertreter: **Paul Hartmann AG
Patents & Licensing
Paul-Hartmann-Straße 12
89522 Heidenheim (DE)**

(56) Entgegenhaltungen:
EP-A1- 1 174 101       EP-B1- 2 848 236
US-A1- 2006 025 735    US-A1- 2010 280 479
US-A1- 2014 100 543    US-A1- 2015 148 769
US-A1- 2020 352 796    US-A1- 2022 192 894
US-B2- 10 687 993

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft einen Hygieneartikel des Pad-Typs für Erwachsene, zum einmaligen Gebrauch für die Aufnahme von Körperausscheidungen, wie eine Inkontinenzeinlage, eine Inkontinenzvorlage oder eine Damenbinde.

Derartige Hygieneartikel werden typischerweise in einen Schrittbereich eines Unterwäschestücks hineingelegt, so dass sie im Gebrauch zwischen den Beinen eines Nutzers zu liegen kommen. Um Körperausscheidungen wie beispielsweise Urin aufnehmen zu können und um ein Verschmutzen des Unterwäschestücks zu vermeiden, umfassen derartige Hygieneprodukte typischerweise ein flüssigkeitsdurchlässiges körperzugewandtes Topsheet und ein dem Unterwäschestück zugewandtes flüssigkeitsundurchlässiges Backsheet, sowie einen zwischen Topsheet und Backsheet sandwichartig angeordneten Absorptionskörper für die dauerhafte Flüssigkeitsspeicherung.

Der Absorptionskörper enthält als absorbierendes Material häufig synthetische oder natürliche Fasern wie Zellstofffasern und/oder superabsorbierendes Material, insbesondere ein superabsorbierendes Polymer (SAP), das eine hohe Absorptionskapazität für Körperflüssigkeiten aufweist. Um eine hohe Absorptionskapazität zu erreichen, wird häufig eine hohe Gesamtmenge an absorbierendem Material oder ein hoher Anteil an SAP eingesetzt. Jedoch hat sich gezeigt, dass auch Hygieneartikel mit einer hohen Absorptionskapazität große Flüssigkeitsmengen oft nicht schnell genug aufnehmen können, und daher eine Gefahr des Auslaufens besteht. Beispielsweise weisen moderne Hygieneartikel häufig einen hohen Anteil an SAP im Absorptionskörper auf, da SAP die gebundene Flüssigkeit auch unter Druck gut binden kann und daher eine Rücknässung vorteilhaft gering ist. Jedoch birgt gequollenes SAP die Gefahr des sogenannten Gelblockings, so dass einmalige große Flüssigkeitsmengen oder ein wiederholter Flüssigkeitsanfall oft nicht schnell genug aufgenommen werden können und auch keine Hohlräume zur Zwischenspeicherung vorhanden sind.

Um ein Auslaufen zu verhindern sind daher im Stand der Technik seitliche Auslaufsperren bekannt, die den Hygieneartikel im Gebrauch in eine dreidimensionale Form bringen und den Hygieneartikel zum Körper hin zumindest teilweise abdichten. Dadurch kann die Flüssigkeit besser im Hygieneartikel gehalten werden, bis sie vom Absorptionskörper aufgesogen ist. EP3716927A1 lehrt einen absorbierenden Hygieneartikel des Pad-Typs in Form eines Bindenprodukts mit Topsheet, Backsheet und Absorptionskörper. Der Hygieneartikel soll im Gebrauch mithilfe bestimmter Faltungen eine Schalenform annehmen, wodurch einem Auslaufen entgegengewirkt werden soll. Der Hygieneartikel weist zwischen Topsheet und Backsheet seitliche elastische Komponenten in Form von bandförmigen Schaumelastiks auf, die zur Formgebung beitragen können. EP2246017A1 lehrt ebenfalls einen absorbierenden Hygieneartikel des Pad-Typs mit Topsheet, Backsheet und Absorptionskörper. Der Hygieneartikel weist seitliche elastische Komponenten auf, die aus einem Schaummaterial, elastischen Fäden oder einem elastischen Filmmaterial ausgebildet sind und die eine spontane Ausbildung einer schalenartigen Formgebung unterstützen. Der Hygieneartikel soll eine reduzierte Gefahr des Auslaufens und einen verbesserten Tragekomfort aufweisen. Elastische Komponenten aus Schaummaterial sollen eine Weichheit des Produktes für den Anwender erhöhen. EP2848236A1 lehrt ein Bindenprodukt mit Beinabschlüssen, welche elastische Elemente aufweisen, die Elasthan- oder Lycrafäden, Schaumstoffstreifen oder elastische Folienstreifen sind, und die Trageeigenschaften verbessern sollen.

Um im Gebrauch eine für das Ausbilden einer schalenartigen Formgebung erforderliche Rückstellkraft der seitlichen elastischen Elemente zu erhalten, werden die elastischen Komponenten häufig in gedehntem Zustand in den Hygieneartikel eingebracht, also in gedehntem Zustand mit anderen, nicht gedehnten Komponenten des Hygieneartikels verbunden, wie beispielsweise in EP2246017A1 und EP2848236A1 beschrieben. Wenn die dehnenden Kräfte weggenommen werden, ziehen sich die elastischen Komponenten wieder zusammen und nehmen einen entspannten Zustand an. Dabei geht der Artikel von einem flach ausgestreckten Zustand in einen in der Gebrauchssituation erwünschten, mehr oder weniger ausgeprägten dreidimensionalen, schalenartig geformten Zustand über.

Um eine Gefahr der Beeinträchtigung der elastischen Eigenschaft vor dem Gebrauch der Hygieneartikel zu mindern, können solche Hygieneartikel herstellerseitig auf sich selbst gefaltet sein. Bei derart gefalteten Artikeln kann es für den Anwender schwierig sein zu erkennen, ob ein Hygieneartikel über eine derartige vorteilhafte Elastifizierung verfügt. Dies ist insbesondere dann der Fall, wenn, wie beispielsweise in einer Pflegeeinrichtung, verschiedene Bindenprodukte mit oft ähnlicher äußerer Erscheinung vorrätig gehalten werden. Auch bei nicht gefalteten Hygieneartikeln, die beispielsweise in einem Vorratsschrank lagern, insbesondere in Stapelform, kann es für den Anwender nicht ohne Weiteres erkennbar sein, ob die vorrätigen Hygieneartikel über eine Elastifizierung verfügen.

Ein weiterer Hygieneartikel ist in US2020/352796 beschrieben.

Ein vorzeitiges Auffalten gefalteter Hygieneartikel und/oder eine häufige In-die-Hand-Nahme gefalteter oder ungefalteter Hygieneartikel sollte jedoch aus Gründen der Hygiene und effektiver Arbeitsabläufe vermieden werden. Eine erleichterte Erkennbarkeit elastifizierter Hygieneartikel ist daher wünschenswert.

Ausgehend vom Stand der Technik war es Aufgabe der Erfindung, einen verbesserten Hygieneartikel mit seitlichen elastischen Elementen bereitzustellen, bei dem das Vorhandensein der seitlichen elastischen Elemente auf hygienische, einfache und unaufwendige Weise festgestellt werden kann.

Die Erfindung löst diese Aufgabe durch einen absorbierenden Hygieneartikel mit den Merkmalen des Anspruchs 1:

Dazu umfasst der absorbierende Hygieneartikel ein auf einer körperzugewandten Seite des Hygieneartikels angeordnetes Topsheet, ein auf einer körperabgewandten Seite des Hygieneartikels angeordnetes Backsheet und einen zwischen dem Topsheet und dem Backsheet angeordneten Absorptionskörper, wobei der Hygieneartikel eine Längsrichtung und eine Querrichtung aufweist, wobei das Topsheet und das Backsheet sich jeweils in der Querrichtung und in der Längsrichtung über den Absorptionskörper hinaus erstrecken und außerhalb des Absorptionskörpers gemeinsam einen Überlappungsbereich bilden, wobei der Hygieneartikel eine Inkontinenzeinlage, eine Inkontinenzvorlage oder eine Damenbinde ist, wobei der Hygieneartikel in der Querrichtung beidseits des Absorptionskörpers jeweils ein in der Längsrichtung erstrecktes und zwischen dem Topsheet und dem Backsheet und zumindest bereichsweise in dem Überlappungsbereich angeordnetes elastisches Flachmaterial aufweist, wobei das jeweilige elastische Flachmaterial gemeinsam mit dem Topsheet und dem Backsheet jeweils eine elastifizierte Zone in dem Überlappungsbereich bildet, wobei das elastische Flachmaterial eine in dem Hygieneartikel dem Topsheet zugewandte topsheetseitige Flachmaterialseite und eine in dem Hygieneartikel dem Backsheet zugewandte backsheetseitige Flachmaterialseite aufweist, wobei die backsheetseitige Flachmaterialseite eine backsheetseitige Flachmaterialfarbe F1 aufweist, wobei das Backsheet in dem Hygieneartikel eine körperzugewandte Backsheetseite und eine körperabgewandte Backsheetseite aufweist, wobei das Backsheet auf der körperabgewandten Backsheetseite eine äußere Backsheetfarbe F2 aufweist, wobei die backsheetseitige Flachmaterialfarbe F1 von der äußeren Backsheetfarbe F2 verschieden ist, wobei das Backsheet eine Opazität von höchstens 90,0 % aufweist, wobei die backsheetseitige Flachmaterialfarbe F1 des elastischen Flachmaterials innerhalb der elastifizierten Zonen von der körperabgewandten Seite des Hygieneartikels betrachtet visuell wahrnehmbar ist,
wobei ein Farbabstand ΔE zwischen (i) einer Farbe F3 der jeweiligen elastifizierten Zone von der körperabgewandten Seite des Hygieneartikels aus betrachtet und (ii) der backsheetseitigen Flachmaterialfarbe F1 des elastischen Flachmaterials einen Wert von höchstens 70,0 aufweist.

Durch die farbige Ausgestaltung des elastischen Flachmaterials erhält der Anwender einen visuell wahrnehmbaren Hinweis auf das Vorhandensein einer zusätzlichen Komponente, mithin einer elastischen Funktion in dem Hygieneartikel, der auf einfache und unaufwendige Weise mit bloßem menschlichen Auge erkennbar, also beispielsweise ohne Einsatz einer manuellen oder instrumentellen Prüfung elastischer Eigenschaften erkennbar ist. Zwar kann ein im gedehnten Zustand in den Hygieneartikel eingebrachtes elastisches Material nach der Wegnahme der dehnenden Kräfte zu einer Kräuselung der umgebenden Materialien wie Backsheet und/oder Topsheet führen. Jedoch können der Kräuselung optisch ähnliche Knicke oder Falten im Überlappungsbereich auch durch Verpackungsschritte wie eine Kompression des Artikels entstehen. Von derlei Knicken oder Falten ist eine Kräuselung durch elastisches Material insbesondere unter nicht optimalen Lichtverhältnissen häufig nur schwer zu unterscheiden - sofern eine solche Kräuselung überhaupt erkennbar ist.

Die Anordnung des elastischen Flachmaterials zwischen Topsheet und Backsheet erlaubt es, im Gebrauch einen direkten Kontakt des farbigen Flachmaterials mit der Haut oder der Kleidung des Trägers zu vermeiden. Dadurch kann einer Übertragung beispielsweise von durch Abrieb gelösten Farbpigmenten vorgebeugt werden.

Das elastische Flachmaterial ist vorzugsweise einfarbig ausgebildet. Es kann aber auch mehrfarbig ausgebildet sein, insbesondere ein mehrfarbiges Muster, beispielsweise ein Streifenmuster, Punktmuster oder Streudekormuster aufweisen.

Der verwendete Begriff "Farbe" oder "farbig" bezieht sich auf die visuelle Wahrnehmungseigenschaft von aus dem Lichtspektrum abgeleiteten und als solche vom Menschen über das Auge erkennbare Lichtleistung. Farben sind typischerweise bekannt wie u.a. Weiß, Schwarz, Rot, Blau, Grün, Gelb und Mischungen davon. Zwei Farben werden im Rahmen dieser Erfindung als voneinander verschieden betrachtet, wenn ihr Farbabstand delta E gemessen nach der nachfolgend beschriebenen Prüfmethode einen Wert größer als 5,0 aufweist.

Das elastische Flachmaterial kann aus einem farbigen und/oder pigmenthaltigen Material ausgebildet oder mit einem farbigen und/oder pigmenthaltigen Material einseitig oder beidseitig beschichtet oder bedruckt sein. Weiter kann das elastische Flachmaterial einschichtig oder mehrschichtig, beispielsweise mittels Koextrusion, ausgebildet sein. Ein mehrschichtiges elastisches Flachmaterial kann Schichten gleichen oder verschiedenen Materials oder gleicher oder verschiedener Zusammensetzung aufweisen. Ein mehrschichtiges elastisches Flachmaterial kann Schichten gleicher oder verschiedener Farbe aufweisen. Weiter kann das elastische Flachmaterial beidseitig, also auf der topsheetseitigen Flachmaterialseite und der backsheetseitigen Flachmaterialseite, die gleiche Farbe oder verschiedene Farben aufweisen.

Als die backsheetseitige Flachmaterialfarbe F1 ist die Farbe des elastischen Flachmaterials als solches im ungedehnten Zustand, also vor einem Einbringen in den Hygieneartikel oder gegebenenfalls nach Entnahme des elastischen Flachmaterials aus dem Hygieneartikel zu Prüfzwecken, mithin im Zustand der natürlichen Ausgangserstreckung des elastischen Flachmaterials ohne Einfluss dehnender Kräfte, zu verstehen.

Als die äußere Backsheetfarbe F2 ist die Farbe des Backsheets als solches, also vor dem Einbringen des Backsheets in den Hygieneartikel oder gegebenenfalls nach Entnahme des Backsheets aus dem Hygieneartikel zu Prüfzwecken, mithin im Zustand der natürlichen Ausgangserstreckung des Backsheets, zu verstehen.

In einer bevorzugten Ausführungsform ist das Backsheet im Wesentlichen weiß. Damit ist gemeint, dass eine vorherrschende Grundfarbe des Backsheets vom Betrachter als weiß wahrgenommen wird. Im Rahmen dieser Erfindung werden als "weiß" neben Reinweiß oder Schneeweiß auch gedeckte Farbnuancen oder Farbnuancen mit einem leichten Farbstich wie beispielsweise cremeweiß, milchweiß, elfenbeinweiß, beigeweiß, altweiß, off-white oder champagnerfarben verstanden. Weiter bevorzugt ist das Backsheet heller als die elastifizierte Zone, insbesondere ist die äußere Backsheetfarbe F2 heller als die Farbe F3 der elastifizierten Zone.

Durch eine eher helle und neutrale vorherrschende Grundfarbe des Backsheets kann auf vorteilhafte Weise eine attraktions- und damit aufmerksamkeitssteigernde Ausgestaltung bereitgestellt und damit dem Anwender das Erkennen der relativ zum Backsheet verschiedenfarbigen elastischen Zone erleichtert werden.

Der Begriff "elastische Zone" ist im Rahmen dieser Erfindung als ein effektiv elastifizierter Teilbereich des Überlappungsbereichs zu verstehen, welcher von dem Topsheet und von dem Backsheet und von dem jeweiligen elastischen Flachmaterial überfangen ist. Im Falle dass das elastische Flachmaterial mit dem Absorptionskörper überlappt, ist ein jeweiliger mit dem Absorptionskörper überlappender Abschnitt des elastischen Flachmaterials für die unten näher beschriebene Bestimmung der Farbe F3 und/oder einer nachfolgend beschriebenen weiteren Farbe F6 der elastifizierten Zone nicht als Teil der elastischen Zone zu betrachten.

Als Farbe F3 der jeweiligen elastifizierten Zone ist die Farbe des intakten Hygieneartikels innerhalb der elastifizierten Zone von der körperabgewandten Seite aus betrachtet zu verstehen. Dabei ist die elastifizierte Zone zu Prüfzwecken gegebenenfalls so weit in der Längsrichtung des Hygieneartikels zu dehnen, dass die im Wesentlichen unelastischen Materialien des Hygieneartikels wie Backsheet und/oder Topsheet bis zu ihrer natürlichen Ausgangserstreckung ohne Elastifizierung ausgebreitet und auf eine ebene Fläche aufgelegt werden können.

Dadurch dass die backsheetseitige Flachmaterialfarbe F1 von der äußeren Backsheetfarbe F2 verschieden ist, kann ein Nutzer das Vorhandensein einer zusätzlichen Komponente, mithin einer Elastifizierung des Hygieneartikels einfach und unaufwendig und ohne den Artikel in die Hand zu nehmen erkennen. Dies fördert auf vorteilhafte Weise einen effektiven Arbeitsablauf und hilft dabei, den Zeitaufwand, beispielsweise bei der Prüfung von Lagerbeständen oder bei Entnahme von Hygieneartikeln aus einem Vorrat, sowie die Gefahr der Kontamination von Hygieneartikeln zu verringern.

Der Farbabstand ΔE von höchstens 70,0 zwischen der Farbe F3 der jeweiligen elastischen Zone und der backsheetseitigen Flachmaterialfarbe F1 verbessert die visuelle Wahrnehmbarkeit des elastischen Flachmaterials von außen durch den Nutzer und vermindert die Gefahr einer Verfälschung der herstellerseitig vorgesehenen Farbgebung des Hinweises auf die elastischen Eigenschaften.

In einem bevorzugten Ausführungsbeispiel ist das Topsheet zumindest bereichsweise flüssigkeitsdurchlässig und umfasst oder besteht aus einem Vliesmaterial oder einem Folienmaterial oder einer Kombination daraus. Das Vliesmaterial kann beispielsweise ein Spinnvlies oder ein Stapelfaservlies sein. Das Backsheet kann insbesondere eine wasserundurchlässige Folie oder ein wasserundurchlässiges Vlies-Folien-Verbundmaterial umfassen oder daraus bestehen. Solche Materialien weisen häufig eine hier vorteilhafte Homogenität auf, so dass der Farbeindruck der elastischen Zone von außerhalb des Hygieneartikels aus betrachtet durch strukturelle oder farbliche Heterogenität des Topsheet-und/oder Backsheetmaterials nicht über Gebühr gestört wird.

Aufgrund der Opazität des Backsheets von höchstens 90,0 % wird die visuelle Wahrnehmbarkeit der backsheetseitigen Flachmaterialfarbe F1 des elastischen Flachmaterials von der körperabgewandten Seite des Hygieneartikels her betrachtet vorteilhaft unterstützt.

In einer bevorzugten Ausführungsform weist das Backsheet eine Opazität von höchstens 85,0 %, insbesondere höchstens 80,0 %, weiter insbesondere höchstens 75,0 % auf.

Bevorzugt beträgt die Opazität des Backsheets mindestens 30,0 %, weiter bevorzugt mindestens 40,0 %, weiter bevorzugt mindestens 50,0 %, weiter bevorzugt mindestens 60,0 %, weiter bevorzugt mindestens 70,0 %. Dadurch kann ein unansehnlicher Eindruck des Hygieneartikels nach Gebrauch, also nach der Aufnahme von Körperausscheidungen von der Backsheetseite her betrachtet reduziert werden. Bei der Entsorgung des gebrauchten Artikels wird so ein Eindruck des Nutzers verbessert, dass der Artikel die Körperausscheidungen auf hygienische Weise sicher einschließt.

In dem Überlappungsbereich sind das Backsheet und das Topsheet direkt oder mittelbar mittels dem Fachmann bekannter Fügemethoden miteinander verbunden, beispielsweise Kleben, Schweißen, Ultraschallschweißen oder Wärmesiegeln. Der Überlappungsbereich ist in der Ebene des Hygieneproduktes im flach ausgebreiteten und gegebenenfalls entfalteten Zustand außerhalb des Absorptionskörpers und an den Absorptionskörper direkt angrenzend verortet und umgibt den Absorptionskörper bevorzugt vollständig entlang einer äußeren Absorptionskörperkante.

Der Absorptionskörper dient der Speicherung von Körperausscheidungen, wie beispielsweise Urin, und umfasst dazu flüssigkeitsabsorbierendes Material. Flüssigkeitsabsorbierende Materialien sind im Stand der Technik hinreichend bekannt und umfassen faserartige und/oder partikelförmige Materialien wie beispielsweise Zellulose, superabsorbierendes Polymer oder Mischungen davon. Der Absorptionskörper kann einlagig oder mehrlagig ausgebildet sein und eine optionale Zwischen- und/oder Hüllschicht aus Vlies-, Folien- oder papierartigem Flachmaterial umfassen.

Der absorbierende Hygieneartikel kann eine Flüssigkeitsaufnahme- und -verteilerlage umfassen, die einer schnellen Aufnahme von Körperausscheidungen und deren Weiterleitung an den Absorptionskörper dient. Die Flüssigkeitsaufnahme- und -verteilerlage ist bevorzugt zwischen dem Topsheet und dem Backsheet angeordnet und kann ein- oder mehrlagig ausgebildet sein. Ein bevorzugtes Material der Flüssigkeitsaufnahme- und - verteilerlage ist ein Vliesmaterial umfassend Fasern natürlichen oder künstlichen Ursprungs. Geeignete Vliesmaterialien sind im Stand der Technik hinreichend beschrieben. Die Fasern können von definierter Länge (Stapelfasern) oder kontinuierlich ("endlos", beispielsweise ein "spunbond" genanntes Spinnvlies) sein oder in-situ ausgebildet werden. Die Fasern künstlichen Ursprungs können aus einem einzelnen Polymer oder einer Polymermischung (Einkomponentenfaser) oder aus mehr als einem einzelnen Polymer und/oder einer Polymermischung (Mehrkomponentenfaser) ausgebildet sein. Das Vliesmaterial kann mittels dem Fachmann bekannter Verfahren verfestigt sein wie zum Beispiel Kalandrierung, Thermobonding, Heißluftverfestigung oder Verfestigung mittels Wasserstrahl.

In einer bevorzugten Ausführungsform ist keine der jeweiligen elastifizierten Zonen von der Flüssigkeitsaufnahme- und -verteilerlage überfangen. Dadurch wird eine Gefahr der Beeinträchtigung des von außerhalb des Hygieneartikels her wahrgenommenen Farbeindrucks der backsheetseitigen Flachmaterialfarbe F1 und/oder der nachfolgend näher beschriebenen topsheetseitigen Flachmaterialfarbe F4 vermindert und deren Wahrnehmbarkeit durch den Nutzer verbessert.

In einer bevorzugten Ausführungsform ist das elastische Flachmaterial ein elastisches Folienmaterial oder ein elastisches Schaummaterial.

Das elastische Schaummaterial kann auf vorteilhafte Weise im Gebrauch einen relativ weichen haptischen Eindruck und dadurch verbesserten Tragekomfort vermitteln. Vorzugsweise umfasst das elastische Schaummaterial Polyurethanschaumstoff oder ist daraus gebildet.

Das elastische Folienmaterial umfasst vorzugsweise Materialien synthetischen Ursprungs oder ist daraus gebildet. Alternativ kann das elastische Folienmaterial Materialien natürlichen Ursprungs, beispielsweise Naturlatex, umfassen oder daraus bestehen. Mittels elastischer Folienmaterialien kann auf vorteilhafte Weise eine relativ hohe Rückstellkraft elastifizierter Abschnitte und daher eine verbesserte Auslaufsicherheit bereitgestellt werden.

Das elastische Folienmaterial weist vorzugsweise ein Flächengewicht von 20-50 g/m$^2$, insbesondere 22-45 g/m$^2$, weiter insbesondere 25-40 g/m$^2$ auf.

Bevorzugt weist das elastische Folienmaterial eine Dicke von 10-50 $\mu$m, insbesondere 15-45 $\mu$m, weiter insbesondere 20-40 $\mu$m auf. Das hat den Vorteil, dass die Farbgebung des elastischen Folienmaterials wie beispielsweise durch in das Material eingemischte Pigmente deutlich visuell wahrnehmbar sein kann bei relativ geringer Verbrauchsmenge und Anteil der farbgebenden Komponenten am Folienmaterial. Daher wird auf vorteilhafte Weise eine Gefahr der Beeinträchtigung der elastischen Eigenschaften des Materials, in diesem Beispiel durch farbgebende Stoffbeimischungen, vermindert sowie Ressourcen geschont. Weiter ist es vorteilhaft, ein nicht zu dickes und/oder schweres Folienmaterial zu verwenden, damit es im Hygieneartikel nicht zu stark aufträgt und der Tragekomfort verbessert sein kann.

In einem bevorzugten Ausführungsbeispiel weist das elastische Flachmaterial eine Quererstreckung von 3 mm bis 20 mm, insbesondere 5-18 mm, weiter insbesondere 10-16 mm auf. Dies bewirkt auf vorteilhafte Weise, dass die elastische Zone ausreichend breit ist, so dass ein Nutzer sie leichter wahrnehmen kann. Dabei trägt das Flachmaterial in der Querrichtung des Hygieneartikels nicht zu stark auf, so dass der Tragekomfort verbessert sein kann.

Bevorzugt weist das elastische Flachmaterial eine Längserstreckung von 90 mm bis 250 mm, insbesondere 100 mm bis 240 mm, weiter insbesondere 110 mm bis 230 mm auf. Dies unterstützt weiter eine gute Wahrnehmbarkeit der Farbgebung und damit des Hinweises auf die Elastifizierung durch den Nutzer, da die elastische Zone in ihrer Längserstreckung besser betont ist. Die Längserstreckung des elastischen Flachmaterials ist hier zu verstehen im gegebenenfalls aufgefalteten und plan ausgebreiteten Hygieneartikel derart, dass die im Wesentlichen unelastischen Materialien des Hygieneartikels wie Backsheet und/oder Topsheet bis zu ihrer natürlichen Ausgangserstreckung ohne Elastifizierung ausgebreitet und auf eine ebene Fläche aufgelegt werden können.

In einer bevorzugten Ausführungsform ist das elastische Flachmaterial jeweils vollständig in dem Überlappungsbereich angeordnet. Dadurch ist das elastische Flachmaterial auf vorteilhafte Weise vollständig von anderen Komponenten des Hygieneartikels wie Topsheet und/oder Backsheet überfangen und kommt nicht direkt mit der Haut des Nutzers in Berührung. Das trägt zu einem verbesserten Tragekomfort bei. Weiter kann eine Gefahr der Beeinträchtigung der elastischen Eigenschaft und der Farbwahrnehmung aufgrund eines Überlappens des elastischen Flachmaterials mit dem Absorptionskörper verringert sein. Weiter kann auch die Integrität des Absorptionskörpers besser erhalten bleiben, da dieser nicht direkt unter dem Einfluss der Elastifizierung steht und dadurch ungewollt verformt werden kann. Es ist jedoch denkbar und möglich, dass das elastische Flachmaterial in einer alternativen Ausführungsform zumindest teilweise mit dem Absorptionskörper überlappt. Solchenfalls ist das elastische Flachmaterial bevorzugt zwischen dem Topsheet und dem Absorptionskörper oder zwischen dem Backsheet und dem Absorptionskörper angeordnet.

Weiter beträgt eine Längserstreckung des elastischen Flachmaterials vorzugsweise mindestens 20 %, insbesondere 20-80 %, weiter insbesondere 30-70 %, weiter insbesondere 40-60 % einer Längserstreckung des Hygieneartikels. Dabei wird eine vorteilhafte körpernahe Formgebung des Hygieneartikels im Gebrauch unterstützt. Zudem ist hierdurch der Hinweis auf die vorhandenen elastischen Eigenschaften des Artikels, insbesondere als bereichsweise Elastifizierung mit Bezug zur Längserstreckung des Artikels im Gegensatz zu einer rein dekorativen und insbesondere über die gesamte Längserstreckung des Artikels hinweg erstreckte Farbgestaltung, besser betont.

Bevorzugt weist der Hygieneartikel entlang der Längsrichtung einen vorderen Bereich, einen hinteren Bereich und einen zwischen dem vorderen Bereich und dem hinteren Bereich angeordneten, zwischen den Beinen eines Nutzers zu liegen kommenden Schrittbereich auf, wobei das elastische Flachmaterial in der Längsrichtung zumindest in dem Schrittbereich angeordnet ist. Das elastische Flachmaterial kann sich optional in den vorderen Bereich und/oder den hinteren Bereich erstrecken. Der Schrittbereich überfängt typischerweise einen Miktionsbereich, also einen Bereich des wahrscheinlichen ersten Kontakts einer Urinausscheidung mit dem Hygieneartikel während einer Miktion. Durch eine solche Anordnung des elastischen Flachmaterials wird eine verbesserte Auslaufsicherheit und vorteilhafte Formgebung des Hygieneartikels im Gebrauch unterstützt, da zumindest der Schrittbereich eine schalenartige Form annehmen kann. Zudem kann hierdurch der Hinweis auf die elastischen Eigenschaften des Artikels, insbesondere mit Bezug zur Elastifizierung des Schrittbereichs besser betont sein. Der vordere Bereich, der hintere Bereich und der Schrittbereich des Hygieneartikels erstrecken sich vorzugsweise jeweils über mindestens 20 %, weiter insbesondere mindestens 25 %, weiter insbesondere mindestens 30 %, weiter insbesondere ein Drittel der Längserstreckung des Hygieneartikels.

Zur Ausbildung seitlicher Längsabschnitte des Überlappungsbereichs kann das Topsheet auf die körperabgewandte Seite des Hygieneartikels umgeschlagen und mit der körperabgewandten Backsheetseite verbunden sein. Hierdurch kann insbesondere im elastifizierten Schrittbereich eine Anmutung der Weichheit verstärkt und der Tragekomfort verbessert werden.

In einem bevorzugten Ausführungsbeispiel weist das Backsheet ein Flächengewicht von 15-35 $g/m^2$, insbesondere 17-30 $g/m^2$, weiter insbesondere 19-28 $g/m^2$ auf. Durch ein Backsheet mit nicht zu geringem Flächengewicht können im gebrauchten Artikel wenig farbkräftige Körperflüssigkeiten wie beispielsweise Urin besser verdeckt und damit der hygienische Eindruck des Nutzers verbessert werden. Gleichzeitig wird bei einem nicht zu hohen Flächengewicht des Backsheets die Wahrnehmbarkeit der backsheetseitigen Flachmaterialfarbe F1 von der körperabgewandten Seite des Hygieneartikels her nicht über Gebühr beeinträchtigt.

In einer bevorzugten Ausführungsform ist der Absorptionskörper vorzugsweise sanduhrförmig ausgebildet. Der Begriff "sanduhrförmig" ist so zu verstehen, dass der Absorptionskörper zumindest in einem zwischen den Beinen eines Nutzers zu liegen kommenden Schrittbereich des Absorptionskörpers in der Querrichtung eine geringere Erstreckung als in einem vorderen Bereich und/oder hinteren Bereich des Absorptionskörpers aufweist. Dadurch kann das in dem Überlappungsbereich angeordnete elastische Flachmaterial auf aufmerksamkeitssteigernde Weise betont sein und damit dem Nutzer das Erkennen erleichtern. Alternativ kann der Absorptionskörper auch rechteckig oder oval ausgebildet sein. Der Absorptionskörper kann weiter in der Längsrichtung symmetrisch oder asymmetrisch ausgebildet sein, also beispielsweise kann der hintere Bereich in der Längsrichtung gleich weit oder weiter oder weniger weit erstreckt sein als der vordere Bereich des Absorptionskörpers.

In einer vorteilhaften Ausführungsform ist der Hygieneartikel ein gefalteter Hygieneartikel, der an mindestens einer in Querrichtung verlaufenden Faltlinie in Richtung der körperzugewandten Seite auf sich selbst gefaltet ist derart, dass das Topsheet zumindest bereichsweise auf sich selbst zu liegen kommt und dass das Backsheet eine erste Oberfläche und eine der ersten Oberfläche entgegengesetzt zu verortende zweite Oberfläche des gefalteten Hygieneartikels bildet, wobei das elastische Flachmaterial sich über die mindestens eine Faltlinie hinweg erstreckt derart, dass das elastische Flachmaterial von der ersten Oberfläche und von der zweiten Oberfläche des gefalteten Hygieneartikels aus betrachtet visuell wahrnehmbar ist. Das ermöglicht eine Wahrnehmung des Hinweises auf die elastischen Eigenschaften des Hygieneartikels von beiden Oberflächen des gefalteten Artikels her, so dass ein Nutzer unabhängig von der betrachteten Oberfläche erkennen kann, dass es sich um einen Artikel mit elastischen Eigenschaften handelt.

In einer weiteren vorteilhaften Ausführungsform weist die topsheetseitige Flachmaterialseite eine topsheetseitige Flachmaterialfarbe F4 auf, wobei das Topsheet in dem Hygieneartikel eine körperzugewandte Topsheetseite und eine körperabgewandte Topsheetseite aufweist, wobei das Topsheet auf der körperzugewandten Topsheetseite eine äußere Topsheetfarbe F5 aufweist, wobei die topsheetseitige Flachmaterialfarbe F4 von der äußeren Topsheetfarbe F5 verschieden ist, wobei das Topsheet eine Opazität von höchstens 90,0 %, insbesondere höchstens 80,0 %, weiter insbesondere höchstens 70,0 %, weiter insbesondere höchstens 60,0 %, weiter insbesondere höchstens 50,0 %, weiter insbesondere höchstens 40,0 % aufweist, wobei die topsheetseitige Flachmaterialfarbe F4 des elastischen Flachmaterials innerhalb der elastifizierten Zonen von der körperzugewandten Seite des Hygieneartikels betrachtet visuell wahrnehmbar ist, wobei die jeweiligen elastifizierten Zonen von der körperzugewandten Seite des Hygieneartikels aus betrachtet eine weitere Farbe F6 aufweisen, wobei ein Farbabstand $\Delta E'$ zwischen (i) der weiteren Farbe F6 und (ii) der topsheetseitigen Flachmaterialfarbe F4 des elastischen Flachmaterials einen Wert von höchstens 50,0 aufweist.

Als die topsheetseitige Flachmaterialfarbe F4 ist die Farbe des elastischen Flachmaterials als solches im ungedehnten Zustand, also vor einem Einbringen in den Hygieneartikel oder gegebenenfalls nach Entnahme des elastischen Flachmaterials aus dem Hygieneartikel zu Prüfzwecken, mithin im Zustand der natürlichen Ausgangserstreckung des elastischen Flachmaterials ohne Einfluss dehnender Kräfte, zu verstehen. Als die äußere Topsheetfarbe F5 ist die Farbe des Topsheets als solches, also vor dem Einbringen des Topsheets in den Hygieneartikel oder gegebenenfalls nach Entnahme des Topsheets aus dem Hygieneartikel zu Prüfzwecken, mithin im Zustand der natürlichen Ausgangserstreckung des Topsheets, zu verstehen.

Als weitere Farbe F6 der jeweiligen elastifizierten Zone ist die Farbe des intakten Hygieneartikels innerhalb der elastifizierten Zone von der körperzugewandten Seite aus betrachtet zu verstehen. Dabei ist die elastifizierte Zone zu Prüfzwecken gegebenenfalls so weit in der Längsrichtung des Hygieneartikels zu dehnen, dass die im Wesentlichen unelastischen Materialien des Hygieneartikels wie Backsheet und/oder Topsheet bis zu ihrer natürlichen Ausgangserstreckung ohne Elastifizierung ausgebreitet und auf eine ebene Fläche aufgelegt werden können.

Dadurch dass die topsheetseitige Flachmaterialfarbe F4 von der äußeren Topsheetfarbe F5 verschieden ist, kann ein Nutzer das Vorhandensein einer zusätzlichen Komponente, mithin einer Elastifizierung des Hygieneartikels einfach und unaufwendig und ohne den Artikel in die Hand zu nehmen erkennen. Dies fördert auf vorteilhafte Weise einen effektiven Arbeitsablauf und hilft dabei, den Zeitaufwand, beispielsweise bei der Prüfung von Lagerbeständen oder bei Entnahme von Hygieneartikeln aus einem Vorrat, sowie die Gefahr der Kontamination von Hygieneartikeln zu verringern. Weiter kann dadurch auch im Gebrauchszustand, also beispielsweise wenn der Hygieneartikel bereits in ein Kleidungsstück eingelegt ist, von der körperzugewandten Seite her der Hinweis auf die elastischen Eigenschaften durch den Nutzer wahrgenommen werden, ohne dass dazu der Artikel aus dem Kleidungsstück entnommen werden muss. Der Nutzer kann dabei in der Gebrauchssituation einen Eindruck höheren Auslaufschutzes durch die Elastifizierung und damit ein verbessertes Sicherheitsgefühl erhalten.

Der Farbabstand $\Delta E'$ von höchstens 50,0 zwischen der weiteren Farbe F6 der jeweiligen elastischen Zone und der topsheetseitigen Flachmaterialfarbe F4 verbessert die visuelle Wahrnehmbarkeit des elastischen Flachmaterials von außen durch den Nutzer und vermindert die Gefahr einer Verfälschung der herstellerseitig vorgesehenen Farbgebung des Hinweises auf die elastischen Eigenschaften.

Aufgrund der Opazität des Topsheets von höchstens 90,0 % wird die visuelle Wahrnehmbarkeit der topsheetseitigen Flachmaterialfarbe F4 des elastischen Flachmaterials von der körperzugewandten Seite des Hygieneartikels her betrachtet vorteilhaft unterstützt.

Bevorzugt beträgt die Opazität des Topsheets mindestens 10,0 %, weiter bevorzugt mindestens 20,0 %, weiter bevorzugt mindestens 25,0 %, weiter bevorzugt mindestens 30,0 %. Dadurch kann ein unansehnlicher Eindruck des Hygieneartikels nach Gebrauch, also nach der Aufnahme von Körperausscheidungen von der Topsheetseite her betrachtet reduziert werden. Bei der Entsorgung des gebrauchten Artikels wird so ein Eindruck des Nutzers verbessert, dass der Artikel die Körperausscheidungen auf hygienische Weise sicher einschließt.

In einem bevorzugten Ausführungsbeispiel ist $\Delta E$ größer als $\Delta E'$, wobei insbesondere ein Verhältnis $\Delta E$ zu $\Delta E'$ einen Wert von 1,1-3,5, insbesondere 1,2-3,0, weiter insbesondere 1,3-2,5, weiter insbesondere 1,4-2,2 aufweist. Dadurch wird einer Verfälschung oder Maskierung des Farbeindrucks gerade von der körperzugewandten Seite her entgegengewirkt. Das ermöglicht in der Gebrauchssituation dem Nutzer, den Hinweis auf die elastischen Eigenschaften deutlicher wahrzunehmen, beispielsweise unter weniger günstigen Beleuchtungsverhältnissen. Es wurde nämlich festgestellt, dass häufig die Beleuchtung von Toilettenräumen, insbesondere nachts, weniger hell eingestellt ist, wohingegen für Lagerbereiche oder Vorratsschränke zur Aufbewahrung von Hygieneartikeln eine hellere Ausleuchtung erwünscht ist. Mitunter kann es auch erleichtert sein, eine gewünschte Positionierung des Artikels in einem Kleidungsstück visuell und einfach zu überprüfen, so dass ein geringerer Farbabstand auf der körperzugewandten Seite hilfreich sein kann.

Nach einer bevorzugten Ausführungsform weist das Topsheet ein Flächengewicht von 5-30 $g/m^2$, insbesondere 10-28 $g/m^2$, weiter insbesondere 15-26 $g/m^2$ auf. Durch ein Topsheet mit nicht zu geringem Flächengewicht können im gebrauchten Artikel wenig farbkräftige Körperflüssigkeiten wie beispielsweise Urin besser verdeckt und damit der hygienische Eindruck für den Nutzer verbessert werden. Gleichzeitig wird durch ein nicht zu hohes Flächengewicht des Topsheets die Wahrnehmbarkeit der topsheetseitigen Flachmaterialfarbe F4 von der körperzugewandten Seite des Hygieneartikels her verbessert. In einer bevorzugten Ausführungsform ist das Topsheet im Wesentlichen weiß. Damit ist gemeint, dass eine vorherrschende Grundfarbe des Topsheets vom Betrachter als weiß wahrgenommen wird. Im Rahmen dieser Erfindung werden als "weiß" neben Reinweiß oder Schneeweiß auch gedeckte Farbnuancen oder Farbnuancen mit einem leichten Farbstich wie beispielsweise cremeweiß, milchweiß, elfenbeinweiß, beigeweiß, altweiß, off-white oder champagnerfarben verstanden. Weiter bevorzugt ist das Topsheet heller als die elastifizierte Zone, insbesondere ist die äußere Topsheetfarbe F5 heller als die weitere Farbe F6 der elastifizierten Zone. Durch eine eher helle und neutrale vorherrschende Grundfarbe des Topsheets kann auf vorteilhafte Weise eine attraktions- und damit aufmerksamkeitssteigernde Ausgestaltung bereitgestellt und damit dem Anwender das Erkennen der relativ zum Topsheet verschiedenfarbigen elastischen Zone erleichtert werden.

Farbmessung nach dem CIEL*a*b*-Farbraum und daraus abgeleitete Farbabstände delta E (auch $\Delta E^*$; im Rahmen dieser Erfindung als Farbabstände $\Delta E$ oder $\Delta E'$ bezeichnet):
Die Farbmessung nach L*a*b*, auch genannt CIEL*a*b*-Farbraum, beschreibt und definiert Farben geräteunabhängig. Der CIEL*a*b*-Farbraum ist genormt in der DIN EN ISO 11664-4: 2012-06 Teil 4.

Der CIEL*a*b*-Farbraum basiert im Wesentlichen auf der Darstellung einer Farbe innerhalb eines Koordinatensystems mit drei Achsen. Die Achse L* repräsentiert die Helligkeit zwischen 0 (schwarz) - 100 (weiß); die a*-Achse gibt den Grün- oder Rotanteil wieder, mit negativen Werten für Grün und positiven Werten für Rot. Die b*-Achse gibt den Blau- oder Gelb-Anteil wieder, dabei mit negativen Werten für Blau und positiven Werten für Gelb.

Prüfgerät

Für die Farbmessungen wird als Prüfgerät ein Spektralphotometer eingesetzt, wie z.B. das Spectroguide sphere gloss von BYK-Gardner.

Probenvorbereitung:

Die Messprobe (Backsheet, Topsheet, elastisches Flachmaterial oder der Hygieneartikel) wird auf einer ebenen Fläche flach ausgebreitet. Für die Vermessung wird jeweils ein Abschnitt des Backsheetmaterials, Topsheetmaterials und/oder des elastischen Flachmaterials in dessen jeweiligem Ausgangszustand vor Einbringen in einen Hygieneartikel verwendet. Alternativ wird zur Vermessung dieser Materialien der Hygieneartikel gegebenenfalls aufgetrennt, um für die Vermessung das jeweilige Material (elastisches Flachmaterial, Backsheet oder Topsheet) vorliegen zu haben. Für die Probengröße ist letztlich die von der Messfeldbegrenzung des Prüfgerätes vorgegebene Messfläche zu beachten.

Die Messungen werden an dem jeweiligen in der Beschreibung genannten Zustand der Messprobe bzw. des Artikels vorgenommen.

- A) Ungedehnt, also im Zustand der natürlichen Ausgangserstreckung des jeweiligen Materials (elastisches Flachmaterial/Backsheet/Topsheet als solches)
- B) Gedehnt (jeweilige elastifizierte Zone des intakten Hygieneartikels), also so weit in der Längsrichtung des Hygieneartikels gedehnt, dass die im Wesentlichen unelastischen Materialien des Hygieneartikels wie Backsheet und/oder Topsheet bis zu ihrer natürlichen Ausgangserstreckung ohne Elastifizierung ausgebreitet und auf eine ebene Fläche aufgelegt werden können.

Für den Messansatz B) wird der Artikel gerade und ohne Verwindung in eine der Längsrichtung des Hygieneartikels entsprechenden Richtung gestreckt, und in diesem Dehnungszustand auf einer dafür geeigneten Unterlage fixiert. Für den Fall, dass die Architektur des Artikels im intakten Zustand eine solche Dehnung nicht zulässt, wird die elastische Zone vor dem Dehnen und Fixieren aus dem Hygieneartikel ausgeschnitten.

Kalibrieren:

Das Prüfgerät ist nach den Herstellervorgaben und dafür vorgesehenen Standards kalibriert.

Messvorgang:

Für die hierbei vorzunehmenden Messungen wird eine standardisierte weiße Testkarte als Referenzfläche unterhalb der Messprobe im Bereich des vorgesehenen Messfeldes gelegt, so dass die jeweiligen Farbmessungen stets gegenüber einer Standardfläche durchgeführt werden.

Eine jeweilige zu vermessende Oberseite der Messprobe ist dabei dem Prüfgerät und dessen optischen Messfeldbereichen zugewandt angeordnet.

Als Referenzfläche werden die für die Opazitätsmessung vorgesehene "opacity charts" von BYK-Gardner, dabei die Karte #2810 eingesetzt (erhältlich bei BYK-Gardner GmbH, Geretsried, Deutschland).

Diese weiße Referenzflächen-Testkarte weist einen L*a*b*-Wert von

L* von 92,12 ± 0,79
a* von -0,95 ± 0,14
b* von 3,69 ± 0,42 auf,
dabei als Mittelwert ± 6 sigma (Standardabweichung)

Für den Messvorgang wird der Messkopf des Gerätes senkrecht und ohne zusätzlichen Druck auf die vorbereitete Messprobe und die gewünschte zu messende Oberseite der Messprobe aufgesetzt. Dafür wird, je nach Konstruktion des Prüfgerätes, zuerst mit einer Komponente die eine Messfeldbegrenzung aufweist (sogenannter Probenbeobachter), die für die Messung relevante Stelle der Messprobe auf der Messprobe festgelegt. Diesem Messfeld gegenüber wird dann das Prüfgerät mit seiner Messöffnung aufgesetzt.

Für das Prüfverfahren und den Messvorgang werden am Prüfgerät als Einstellungen vorgenommen:

- Skala: CIEL*a*b*
- Index: ΔE*
- Lichtart: D65
- Winkel: 10°
- Messöffnung: 11 mm

Im Falle von dazu schmaleren Messbereichen ist ein Prüfgerät mit einer entsprechend schmäleren Messöffnung zu wählen

Es werden von einer Messprobe n = 6 Messungen vorgenommen.

Dabei wird die Messung an verschiedenen Stellen durchgeführt.

Auswertung:

Aus den Messwerten wird jeweils der Mittelwert für L*, a* und b* sowie für die Opazität ermittelt.

Die Messwerte und der Mittelwert werden auf eine Dezimale nach dem Komma angegeben. Die Farbtöne werden als L*, a*, b* Wert angegeben. Der Wert ist dimensionslos. Die Opazität wird in Prozent [%] angegeben.

Für den Vergleich von Farbmessungen an verschiedenen Bereichen bzw. verschiedenen Messproben wird der Farbabstand bei Farbmessungen nach dem CIEL*a*b*-Farbraum als delta E (auch ΔE*, im Rahmen dieser Erfindung als Farbabstände ΔE oder ΔE' bezeichnet) wie folgt berechnet:

- für die jeweilige Differenz von jeder der drei Achsen des CIEL*a*b*-Farbraums das Quadrat berechnen
- davon die Summe und über diese Summe die Wurzel berechnen:

$$\Delta E^* = [\,(\Delta L^*)^2 + (\Delta a^*)^2 + (\Delta b^*)^2\,]^{0,5}$$

Der Wert ist dimensionslos.

Berechnung für die Unterschiede in den L*-Werten (Helligkeit):

Für den Vergleich von Helligkeiten werden von aus den Farbmessungen nach dem CIEL*a*b*-Farbraum erhaltenen Ergebnisse der L*- Koordinate die Unterschiede (ΔL*) wie folgt berechnet:

Als einfache Differenzmessung zwischen zwei L*-Werten:

$$\Delta L^* = L^*1 - L^*2.$$

Der Wert ist dimensionslos. Als Betrag angegeben wird der Unterschied als solcher beschrieben. Andernfalls kann damit die Richtung der Helligkeitsverschiebung beschrieben werden.

Weitere Einzelheiten, Merkmale und Vorteile der Erfindung ergeben sich aus den beigefügten Patentansprüchen und aus der zeichnerischen Darstellung und nachfolgenden Beschreibung einer bevorzugten Ausführungsform der Erfindung.

**In der Zeichnung zeigen:**

**Figur 1** schematisch, nicht maßstäblich eine Aufsicht auf einen beispielhaften absorbierenden Hygieneartikel,

**Figur 2** schematisch, nicht maßstäblich einen Querschnitt A-A durch den in Figur 1 dargestellten absorbierenden Hygieneartikel,

**Figuren 3a und 3b** schematisch, nicht maßstäblich jeweils eine Schnittansicht durch einen beispielhaften absorbierenden Hygieneartikel in einem jeweiligen Schrittbereich und

**Figur 4** schematisch, nicht maßstäblich, perspektivisch den beispielhaften absorbierenden Hygieneartikel der Figur 1 in einem gefalteten Zustand.

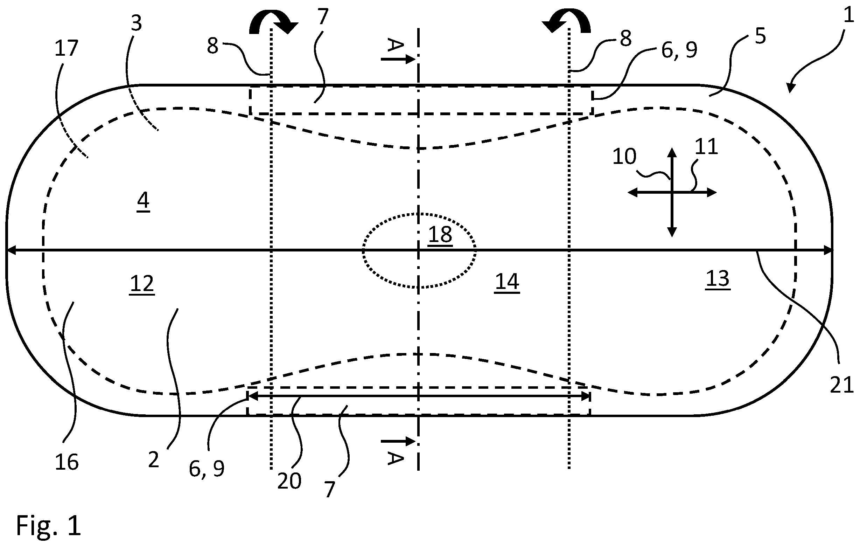

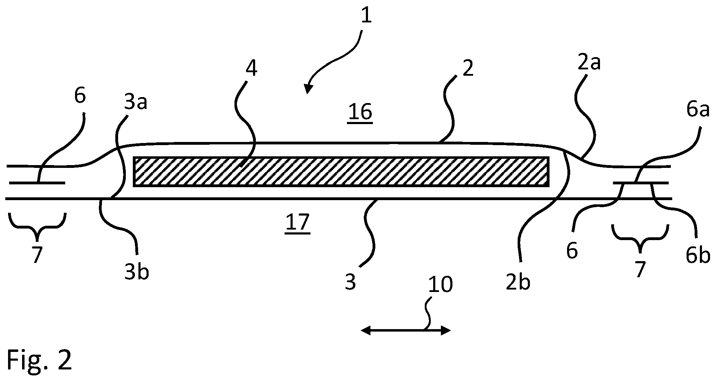

**Figur 1** zeigt schematisch, nicht maßstäblich eine Draufsicht auf einen beispielhaften absorbierenden Hygieneartikel 1 mit einem auf einer körperzugewandten Seite 16 des Hygieneartikels 1 angeordneten Topsheet 2, einem auf einer körperabgewandten Seite 17 des Hygieneartikels 1 angeordneten Backsheet 3 und einem zwischen dem Topsheet 2 und dem Backsheet 3 angeordneten Absorptionskörper 4. Der Hygieneartikel 1 weist eine Längsrichtung 11 und eine Querrichtung 10 auf. Das Topsheet 2 und das Backsheet 3 erstrecken sich jeweils in der Querrichtung 10 und in der Längsrichtung 11 über den Absorptionskörper 4 hinaus und bilden außerhalb des Absorptionskörpers 4 gemeinsam einen Überlappungsbereich 5. Der Hygieneartikel 1 weist in der Querrichtung 10 beidseits des Absorptionskörpers 4 jeweils ein in der Längsrichtung 11 erstrecktes und zwischen dem Topsheet 2 und dem Backsheet 3 und zumindest bereichsweise in dem Überlappungsbereich 5 angeordnetes elastisches Flachmaterial 6 auf. Das jeweilige elastische Flachmaterial 6 bildet gemeinsam mit dem Topsheet 2 und dem Backsheet 3 jeweils eine elastifizierte Zone 7 in dem Überlappungsbereich 5. Das elastische Flachmaterial 6 weist wie in **Figur 2** näher beschrieben eine in dem Hygieneartikel 1 dem Topsheet 2 zugewandte topsheetseitige Flachmaterialseite 6a und eine in dem Hygieneartikel 1 dem Backsheet 3 zugewandte backsheetseitige Flachmaterialseite 6b auf. Dabei weist die backsheetseitige Flachmaterialseite 6b eine backsheetseitige Flachmaterialfarbe F1 auf.

Das Backsheet 3 in dem Hygieneartikel 1 weist eine körperzugewandte Backsheetseite 3a und eine körperabgewandte Backsheetseite 3b auf, wobei das Backsheet 3 auf der körperabgewandten Backsheetseite 3b eine äußere Backsheetfarbe F2 aufweist. Die backsheetseitige Flachmaterialfarbe F1 ist von der äußeren Backsheetfarbe F2 verschieden. Das Backsheet 3 ist in diesem beispielhaften Hygieneartikel 1 ein Vlies-Folien-Verbundmaterial mit einer Opazität von 71,7 %. Alternativ wäre denkbar, ein Folienmaterial als Backsheet vorzusehen.

Die backsheetseitige Flachmaterialfarbe F1 des elastischen Flachmaterials 6 innerhalb der elastifizierten Zonen 7 ist von der körperabgewandten Seite 17 des Hygieneartikels 1 betrachtet visuell wahrnehmbar. Der Hygieneartikel 1 weist einen Farbabstand ∆E zwischen (i) einer Farbe F3 der jeweiligen elastifizierten Zone 7 von der körperabgewandten Seite 17 des Hygieneartikels 1 aus betrachtet und (ii) der backsheetseitigen Flachmaterialfarbe F1 des elastischen Flachmaterials 6 auf mit einem Wert für den Farbabstand ∆E von 54,3.

Der in **Figur 1 und Figur 2** dargestellte Hygieneartikel 1 ist ein Pad-artiger Hygieneartikel für Erwachsene zum einmaligen Gebrauch für die Aufnahme von Körperausscheidungen, wie eine Inkontinenzeinlage, eine Inkontinenzvorlage oder eine Damenbinde.

Derartige Hygieneartikel werden typischerweise in einen Schrittbereich eines Unterwäschestücks hineingelegt, so dass sie im Gebrauch zwischen den Beinen eines Nutzers zu liegen kommen. Optional kann der Hygieneartikel hierzu auf der körperabgewandten Seite Haft- oder Befestigungsmittel aufweisen wie beispielsweise Klebemittel oder andere dem Fachmann als geeignet erscheinende Befestigungsmittel (nicht dargestellt). Der in **Figur 1** dargestellte Hygieneartikel 1 ist in einem flach ausgebreiteten Zustand gezeigt. Der Hygieneartikel 1 kann eine oder mehrere optionale Faltlinien 8 aufweisen, an denen der Hygieneartikel auf sich selbst gefaltet werden kann. In **Figur 1** sind beispielhaft zwei optionale Faltlinien 8 eingezeichnet.

Das elastische Flachmaterial 6 des Hygieneartikels 1 weist eine Quererstreckung von 13 mm und eine Längserstreckung von 145 mm im flach ausgebreiteten Hygieneartikel 1 auf. In diesem Beispiel ist das elastische Flachmaterial 6 ein grünes elastisches Folienmaterial mit einer Dicke von 38 $\mu$m und einem Flächengewicht von 38 g/m$^2$ (zu beziehen vom Hersteller RKW, WB0004.18). Alternativ kann auch ein elastisches Schaummaterial als elastisches Flachmaterial vorgesehen sein.

Das elastische Flachmaterial 6 ist jeweils vollständig in dem Überlappungsbereich 5 angeordnet. Alternativ kann das elastische Flachmaterial auch teilweise, beispielsweise an einem in Längsrichtung endständigen Abschnitt des elastischen Flachmaterials, mit dem Absorptionskörper überlappen. Solchenfalls ist ein jeweiliger mit dem Absorptionskörper überlappender Abschnitt des elastischen Flachmaterials im Rahmen der beschriebenen Farbmessungen nicht als Teil der elastischen Zone zu betrachten. In diesem Beispiel ist der Absorptionskörper 4 sanduhrförmig und in der Längsrichtung symmetrisch ausgebildet (**Figur 1**). Eine rechteckige oder ovale Form oder jede andere dem Fachmann als geeignet erscheinende Form, ist jedoch ebenfalls denkbar. Der Absorptionskörper kann jeweils in der Längsrichtung symmetrisch oder asymmetrisch ausgebildet sein, also beispielsweise kann der hintere Bereich in der Längsrichtung gleich weit oder weiter oder weniger weit erstreckt sein als der vordere Bereich.

Der in **Figur 1** dargestellte Hygieneartikel 1 weist entlang der Längsrichtung einen vorderen Bereich 12, einen hinteren Bereich 13 und einen zwischen dem vorderen Bereich 12 und dem hinteren Bereich 13 angeordneten, zwischen den Beinen eines Nutzers zu liegen kommenden Schrittbereich 14 auf. Das elastische Flachmaterial 6 ist in der Längsrichtung in dem Schrittbereich 14 angeordnet. Das elastische Flachmaterial kann sich optional in den vorderen Bereich und/oder den hinteren Bereich erstrecken. Der Schrittbereich 14 überfängt einen Miktionsbereich 18, also einen Bereich des wahrscheinlichen ersten Kontakts einer Urinausscheidung mit dem Hygieneartikel während einer Miktion. In diesem Beispiel beträgt eine Längserstreckung 20 des elastischen Flachmaterials 6 42 % einer Längserstreckung 21 des Hygieneartikels 1, gemessen im flach ausgebreiteten Zustand. Das Backsheet 3 besteht in diesem Beispiel aus einem wasserundurchlässigen im Wesentlichen weißen Vlies-Folien-Verbundmaterial mit einem Flächengewicht von 24 g/m$^2$. Ein geeignetes Vlies-Folien-Verbundmaterial ist beispielsweise als "Breathable textile backsheet" bestehend aus einem "Spunbond"-Spinnvlies aus Polypropylen und einer Polyethylen-Folie mit einem Flächengewicht von 28 g/m$^2$ vom Hersteller Plastik Textiles erhältlich. Das Backsheet aus Vlies-Folien-Verbundmaterial bietet gegenüber einem Folienmaterial den Vorteil geringerer Geräuschentwicklung im Gebrauch und eines angenehmeren haptischen Eindrucks auf einer von dem Vlies gebildeten körperabgewandten Außenseite des Artikels. In einer alternativen Ausführung des beispielhaften Hygieneartikels besteht das Backsheet aus einer wasserundurchlässigen im Wesentlichen weißen Folie ausgebildet aus dem Polyolefin Polyethylen und mit einem Flächengewicht von 20 g/m$^2$. Ein aus Folienmaterial gebildetes Backsheet kann neben prozesstechnischen Vorteilen und Kostenvorteilen dem Nutzer einen verbesserten Eindruck der Auslaufsicherheit beispielsweise im Vergleich zu einem Material mit textiler Anmutung geben.

In dem in **Figur 1 und Figur 2** dargestellten Hygieneartikel ist der Absorptionskörper einlagig ausgebildet.

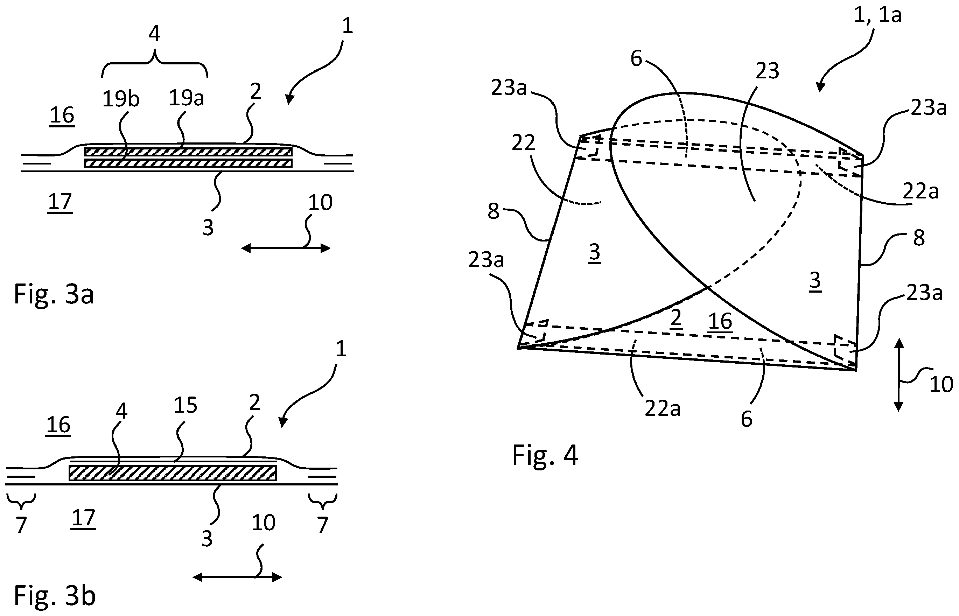

Alternativ kann der Absorptionskörper 4 wie in **Figur 3a** schematisch, nicht maßstäblich dargestellt mehrlagig ausgebildet sein. In dem in **Figur 3a** dargestellte beispielhaften Hygieneartikel 1 umfasst der Absorptionskörper 4 eine zur körperzugewandten Seite 16 angeordnete erste Absorptionskörperlage 19a und eine zur körperabgewandten Seite 17 angeordnete zweite Absorptionskörperlage 19b. Die erste Absorptionskörperlage 19a und die zweite Absorptionskörperlage 19b sind in diesem Beispiel gleich weit in der Querrichtung 10 und in der Längsrichtung (nicht dargestellt) erstreckt. Alternativ können die erste Absorptionskörperlage und die zweite Absorptionskörperlage in der Querrichtung und/oder der Längsrichtung unterschiedlich weit erstreckt sein. Die erste Absorptionskörperlage und die zweite Absorptionskörperlage können eine gleiche oder eine unterschiedliche Zusammensetzung an absorbierenden Materialien wie Zellulose, superabsorbierendes Polymer oder Mischungen davon umfassen.

In **Figur 3b** umfasst der Hygieneartikel 1 eine optionale Flüssigkeitsaufnahme- und -verteilerlage 15. Die Flüssigkeitsaufnahme- und -verteilerlage 15 und der Absorptionskörper 4 sind in diesem Beispiel gleich weit in der Querrichtung 10 und in der Längsrichtung (nicht dargestellt) erstreckt. Keine der elastifizierten Zonen 7 ist von der Flüssigkeitsaufnahme- und -verteilerlage 15 überfangen. Alternativ können die Flüssigkeitsaufnahme- und -verteilerlage und der Absorptionskörper in der Querrichtung und/oder der Längsrichtung unterschiedlich weit erstreckt sein. Die Flüssigkeitsaufnahme- und -verteilerlage 15 besteht in diesem Beispiel aus einem Vliesmaterial bestehend aus Stapelfasern künstlichen Ursprungs und ist zwischen dem Topsheet 2 und dem Backsheet 3, dabei zwischen dem Topsheet 2 und dem Absorptionskörper 4 angeordnet. Alternativ kann die Flüssigkeitsaufnahme- und -verteilerlage auch zwischen dem Absorptionskörper und dem Backsheet angeordnet sein.

In **Figur 4** ist der beispielhafte Hygieneartikel 1 aus **Figur 1** in gefaltetem Zustand dargestellt. Dazu ist der Hygieneartikel 1 an zwei in Querrichtung 10 verlaufenden Faltlinien 8 in Richtung der körperzugewandten Seite 16 auf sich selbst gefaltet. Das Topsheet 2 kommt bereichsweise auf sich selbst zu liegen. Das Backsheet 3 bildet eine erste Oberfläche 22 und eine der ersten Oberfläche 22 entgegengesetzt zu verortende zweite Oberfläche 23 des gefalteten Hygieneartikels 1a. Das elastische Flachmaterial 6 erstreckt sich über die jeweilige Faltlinie 8 hinweg, so dass das elastische Flachmaterial 6 von der ersten Oberfläche 22, 22a und von der zweiten Oberfläche 23, 23a des gefalteten Hygieneartikels 1a aus betrachtet visuell wahrnehmbar ist. Alternativ ist auch denkbar, dass der Hygieneartikel nur eine Faltlinie oder mehr als zwei Faltlinien aufweist, und im gefalteten Zustand von der ersten Oberfläche und von der der ersten Oberfläche entgegengesetzt zu verortenden zweiten Oberfläche jeweils das elastische Flachmaterial visuell wahrnehmbar ist. Alternativ ist denkbar, dass der Hygieneartikel so gefaltet ist, dass nur von einer der ersten Oberfläche oder der zweiten Oberfläche das elastische Flachmaterial visuell wahrnehmbar ist. Der Absorptionskörper und der Überlappungsbereich sind aus Gründen der Übersichtlichkeit in **Figur 4** nicht dargestellt.

Die in **Figur 2** dargestellte topsheetseitige Flachmaterialseite 6a weist in dem beispielhaften Hygieneartikel 1 eine topsheetseitige Flachmaterialfarbe F4 auf, wobei das Topsheet 2 in dem Hygieneartikel 1 eine körperzugewandte Topsheetseite 2a und eine körperabgewandte Topsheetseite 2b aufweist. Das Topsheet 2 weist auf der körperzugewandten Topsheetseite 2a eine äußere Topsheetfarbe F5 auf und die topsheetseitige Flachmaterialfarbe F4 ist von der äußeren Topsheetfarbe F5 verschieden. Das Topsheet 2 ist in diesem beispielhaften Hygieneartikel 1 ein flüssigkeitsdurchlässiges Vliesmaterial mit einer Opazität von 36,2 %. Das Vliesmaterial ist ein im Wesentlichen weißes Spinnvlies ("spunbond") des Herstellers PFN mit einem Flächengewicht von 25 g/m$^2$ und bestehend aus dem Polyolefin Polypropylen. Das Vliesmaterial bietet den Vorteil geringer Geräuschentwicklung im Gebrauch und eines angenehmen und/oder weichen haptischen Eindrucks mit textiler Anmutung auf einer von dem Vlies gebildeten körperzugewandten Außenseite des Artikels. Alternativ wäre denkbar, ein Folienmaterial oder eine Kombination aus Vlies und Folie als Topsheet vorzusehen.

Die topsheetseitige Flachmaterialfarbe F4 des elastischen Flachmaterials 6 des beispielhaften Hygieneartikels 1 ist innerhalb der elastifizierten Zonen 7 von der körperzugewandten Seite 16 des Hygieneartikels 1 betrachtet visuell wahrnehmbar. Die jeweiligen elastifizierten Zonen 7 weisen von der körperzugewandten Seite 16 des Hygieneartikels 1 aus betrachtet eine weitere Farbe F6 auf. Ein Farbabstand ΔE' zwischen (i) der weiteren Farbe F6 und (ii) der topsheetseitigen Flachmaterialfarbe F4 des elastischen Flachmaterials 6 weist einen Wert von höchstens 37,4 auf.

In diesem beispielhaften Hygieneartikel 1 ist ΔE größer als ΔE'. Ein Verhältnis ΔE zu ΔE' weist einen Wert von 1,5 auf.

## Patentansprüche

1.  Absorbierender Hygieneartikel (1) umfassend ein auf einer körperzugewandten Seite (16) des Hygieneartikels (1) angeordnetes Topsheet (2), ein auf einer körperabgewandten Seite (17) des Hygieneartikels (1) angeordnetes Backsheet (3) und einen zwischen dem Topsheet (2) und dem Backsheet (3) angeordneten Absorptionskörper (4), wobei der Hygieneartikel (1) eine Längsrichtung (11) und eine Querrichtung (10) aufweist, wobei das Topsheet (2) und das Backsheet (3) sich jeweils in der Querrichtung (10) und in der Längsrichtung (11) über den Absorptionskörper (4) hinaus erstrecken und außerhalb des Absorptionskörpers (4) gemeinsam einen Überlappungsbereich (5) bilden, wobei der Hygieneartikel (1) eine Inkontinenzeinlage, eine Inkontinenzvorlage oder eine Damenbinde ist,

wobei der Hygieneartikel (1) in der Querrichtung (10) beidseits des Absorptionskörpers (4) jeweils ein in der Längsrichtung (11) erstrecktes und zwischen dem Topsheet (2) und dem Backsheet (3) und zumindest bereichsweise in dem Überlappungsbereich (5) angeordnetes elastisches Flachmaterial (6) aufweist, wobei das jeweilige elastische Flachmaterial (6) gemeinsam mit dem Topsheet (2) und dem Backsheet (3) jeweils eine elastifizierte Zone (7) in dem Überlappungsbereich (5) bildet,

wobei das elastische Flachmaterial (6) eine in dem Hygieneartikel (1) dem Topsheet (2) zugewandte topsheetseitige Flachmaterialseite (6a) und eine in dem Hygieneartikel (1) dem Backsheet (3) zugewandte backsheetseitige Flachmaterialseite (6b) aufweist, wobei die backsheetseitige Flachmaterialseite (6b) eine backsheetseitige Flachmaterialfarbe F1 aufweist,

wobei das Backsheet (3) in dem Hygieneartikel (1) eine körperzugewandte Backsheetseite (3a) und eine körperabgewandte Backsheetseite (3b) aufweist, wobei das Backsheet (3) auf der körperabgewandten Backsheetseite (3b) eine äußere Backsheetfarbe F2 aufweist, wobei die backsheetseitige Flachmaterialfarbe F1 von der äußeren Backsheetfarbe F2 verschieden ist,

wobei das Backsheet (3) eine Opazität von höchstens 90,0 % aufweist, wobei die backsheetseitige Flachmaterialfarbe F1 des elastischen Flachmaterials (6) innerhalb der elastifizierten Zonen (7) von der körperabgewandten Seite (17) des Hygieneartikels (1) betrachtet visuell wahrnehmbar ist,

wobei ein Farbabstand ΔE zwischen (i) einer Farbe F3 der jeweiligen elastifizierten Zone (7) von der körperabgewandten Seite (17) des Hygieneartikels (1) aus betrachtet und (ii) der backsheetseitigen Flachmaterialfarbe F1 des elastischen Flachmaterials (6) einen Wert von höchstens 70,0 aufweist.

2. Absorbierender Hygieneartikel nach Anspruch 1 **dadurch gekennzeichnet, dass** das Backsheet (3) eine Opazität von höchstens 85,0 %, insbesondere höchstens 80,0 %, weiter insbesondere höchstens 75,0 % aufweist.

3. Absorbierender Hygieneartikel nach einem der vorhergehenden Ansprüche **dadurch gekennzeichnet, dass** das elastische Flachmaterial (6) ein elastisches Folienmaterial (9) oder ein elastisches Schaummaterial ist.

4. Absorbierender Hygieneartikel nach Anspruch 3 **dadurch gekennzeichnet, dass** das elastische Folienmaterial eine Dicke von 10-50 μm, insbesondere 15-45 μm, weiter insbesondere 20-40 μm aufweist.

5. Absorbierender Hygieneartikel nach einem der vorhergehenden Ansprüche **dadurch gekennzeichnet, dass** das elastische Flachmaterial (6) eine Quererstreckung von 3 mm bis 20 mm, insbesondere 5-18 mm, weiter insbesondere 10-16 mm aufweist.

6. Absorbierender Hygieneartikel nach einem der vorhergehenden Ansprüche **dadurch gekennzeichnet, dass** das elastische Flachmaterial (6) eine Längserstreckung von 90 mm bis 250 mm, insbesondere 100 mm bis 240 mm, weiter insbesondere 110 mm bis 230 mm aufweist.

7. Absorbierender Hygieneartikel nach einem der vorhergehenden Ansprüche **dadurch gekennzeichnet, dass** das elastische Flachmaterial (6) jeweils vollständig in dem Überlappungsbereich (5) angeordnet ist.

8. Absorbierender Hygieneartikel nach einem der vorhergehenden Ansprüche **dadurch gekennzeichnet, dass** eine Längserstreckung (20) des elastischen Flachmaterials (6) mindestens 20 %, insbesondere 20-80 %, weiter insbesondere 30-70 %, weiter insbesondere 40-60 % einer Längserstreckung (21) des Hygieneartikels (1) beträgt.

9. Absorbierender Hygieneartikel nach einem der vorhergehenden Ansprüche **dadurch gekennzeichnet, dass** der Hygieneartikel (1) entlang der Längsrichtung (11) einen vorderen Bereich (12), einen hinteren Bereich (13) und einen zwischen dem vorderen Bereich (12) und dem hinteren Bereich (13) angeordneten, zwischen den Beinen eines Nutzers zu liegen kommenden Schrittbereich (14) aufweist, wobei das elastische Flachmaterial (6) in der Längsrichtung (11) zumindest in dem Schrittbereich (14) angeordnet ist.

10. Absorbierender Hygieneartikel nach einem der vorhergehenden Ansprüche **dadurch gekennzeichnet, dass** das Backsheet (3) ein Flächengewicht von 15-35 g/m$^2$, insbesondere 17-30, weiter insbesondere 19-28 g/m$^2$ aufweist.

11. Absorbierender Hygieneartikel nach einem der vorhergehenden Ansprüche **dadurch gekennzeichnet, dass** das Backsheet (3) aus einer wasserundurchlässigen Folie oder einem wasserundurchlässigen Vlies-Folien-Verbundmaterial besteht.

**12.** Absorbierender Hygieneartikel nach einem der vorhergehenden Ansprüche **dadurch gekennzeichnet, dass** das Backsheet (3) im Wesentlichen weiß ist.

**13.** Absorbierender Hygieneartikel nach einem der vorhergehenden Ansprüche **dadurch gekennzeichnet, dass** der Absorptionskörper (4) sanduhrförmig ausgebildet ist.

**14.** Absorbierender Hygieneartikel nach einem der vorhergehenden Ansprüche **dadurch gekennzeichnet, dass** der Hygieneartikel (1) ein gefalteter Hygieneartikel (1a) ist, der an mindestens einer in Querrichtung (10) verlaufenden Faltlinie (8) in Richtung der körperzugewandten Seite (16) auf sich selbst gefaltet ist derart, dass das Topsheet (2) zumindest bereichsweise auf sich selbst zu liegen kommt und dass das Backsheet (3) eine erste Oberfläche (22) und eine der ersten Oberfläche (22) entgegengesetzt zu verortende zweite Oberfläche (23) des gefalteten Hygieneartikels (1a) bildet, wobei das elastische Flachmaterial (6) sich über die mindestens eine Faltlinie (8) hinweg erstreckt derart, dass das elastische Flachmaterial (6) von der ersten Oberfläche (22) und von der zweiten Oberfläche (23) des gefalteten Hygieneartikels (1a) aus betrachtet visuell wahrnehmbar ist.

**15.** Absorbierender Hygieneartikel nach einem der vorhergehenden Ansprüche **dadurch gekennzeichnet, dass** die topsheetseitige Flachmaterialseite (6a) eine topsheetseitige Flachmaterialfarbe F4 aufweist, wobei das Topsheet (2) in dem Hygieneartikel (1) eine körperzugewandte Topsheetseite (2a) und eine körperabgewandte Topsheetseite (2b) aufweist, wobei das Topsheet (2) auf der körperzugewandten Topsheetseite (2a) eine äußere Topsheetfarbe F5 aufweist, wobei die topsheetseitige Flachmaterialfarbe F4 von der äußeren Topsheetfarbe F5 verschieden ist, wobei das Topsheet (2) eine Opazität von höchstens 90,0 %, insbesondere höchstens 80,0 %, weiter insbesondere höchstens 70,0 %, weiter insbesondere höchstens 60,0 %, weiter insbesondere höchstens 50,0 %, weiter insbesondere höchstens 40,0 % aufweist, wobei die topsheetseitige Flachmaterialfarbe F4 des elastischen Flachmaterials (6) innerhalb der elastifizierten Zonen (7) von der körperzugewandten Seite (16) des Hygieneartikels (1) betrachtet visuell wahrnehmbar ist, wobei die jeweiligen elastifizierten Zonen (7) von der körperzugewandten Seite (16) des Hygieneartikels (1) aus betrachtet eine weitere Farbe F6 aufweisen, wobei ein Farbabstand ΔE' zwischen (i) der weiteren Farbe F6 und (ii) der topsheetseitigen Flachmaterialfarbe F4 des elastischen Flachmaterials (6) einen Wert von höchstens 50,0 aufweist.

**16.** Absorbierender Hygieneartikel nach Anspruch 15 **dadurch gekennzeichnet, dass** ΔE größer ist als ΔE', wobei insbesondere ein Verhältnis ΔE zu ΔE' einen Wert von 1,1-3,5, insbesondere 1,2-3,0, weiter insbesondere 1,3-2,5, weiter insbesondere 1,4-2,2 aufweist.

**17.** Absorbierender Hygieneartikel nach einem der vorhergehenden Ansprüche **dadurch gekennzeichnet, dass** das Topsheet (2) zumindest bereichsweise flüssigkeitsdurchlässig ist und ein Vliesmaterial oder ein Folienmaterial oder eine Kombination daraus umfasst oder daraus besteht.

**18.** Absorbierender Hygieneartikel nach einem der vorhergehenden Ansprüche **dadurch gekennzeichnet, dass** das Topsheet (2) ein Flächengewicht von 5-30 g/m², insbesondere 10-28 g/m², weiter insbesondere 15-26 g/m² aufweist.

**19.** Absorbierender Hygieneartikel nach einem der vorhergehenden Ansprüche **dadurch gekennzeichnet, dass** das Topsheet (2) im Wesentlichen weiß ist.

**Claims**

**1.** Absorbent hygiene article (1) comprising a topsheet (2) arranged on a side (16) of the hygiene article (1) facing towards the body, a backsheet (3) arranged on a side (17) of the hygiene article (1) facing away from the body, and an absorption body (4) arranged between the topsheet (2) and the backsheet (3), wherein the hygiene article (1) has a longitudinal direction (11) and a transverse direction (10), wherein the topsheet (2) and the backsheet (3) each extend in the transverse direction (10) and in the longitudinal direction (11) beyond the absorption body (4) and together form an overlap region (5) outside the absorption body (4), wherein the hygiene article (1) is an incontinence liner, an incontinence pad or a sanitary pad,

wherein the hygiene article (1) in the transverse direction (10) on both sides of the absorption body (4) has in each case an elastic flat material (6) extending in the longitudinal direction (11) and arranged between the topsheet (2) and the backsheet (3) and at least partially in the overlap region (5), wherein the respective elastic flat material (6) together with the topsheet (2) and the backsheet (3) in each case forms an elastified zone (7) in the overlap region (5), wherein the elastic flat material (6) has a topsheet-side flat material side (6a) facing towards the topsheet (2) in the hygiene article (1) and a backsheet-side flat material side (6b) facing towards the backsheet (3) in the hygiene article (1), wherein the backsheet-side flat material side (6b) has a backsheet-side flat material colour F1, wherein the backsheet (3) in the hygiene article (1) has a backsheet side (3a) facing towards the body and a backsheet side (3b) facing away from the body, wherein the backsheet (3) has, on the backsheet side (3b) facing away from the body, an outer backsheet colour F2, wherein the backsheet-side flat material colour F1 is different from the outer backsheet colour F2,

wherein the backsheet (3) has an opacity of at most 90.0%, wherein the backsheet-side flat material colour F1 of the elastic flat material (6) within the elastified zones (7) is visually perceptible when viewed from the side (17) of the hygiene article (1) facing away from the body,

wherein a colour distance ΔE between (i) a colour F3 of the respective elastified zone (7) when viewed from the side (17) of the hygiene article (1) facing away from the body and (ii) the backsheet-side flat material colour F1 of the elastic flat material (6) has a value of at most 70.0.

2. Absorbent hygiene article according to Claim 1, **characterized in that** the backsheet (3) has an opacity of at most 85.0%, in particular at most 80.0%, more particularly at most 75.0%.

3. Absorbent hygiene article according to either of the preceding claims, **characterized in that** the elastic flat material (6) is an elastic film material (9) or an elastic foam material.

4. Absorbent hygiene article according to Claim 3, **characterized in that** the elastic film material has a thickness of 10-50 $\mu$m, in particular 15-45 $\mu$m, more particularly 20-40 $\mu$m.

5. Absorbent hygiene article according to one of the preceding claims, **characterized in that** the elastic flat material (6) has a transverse extent of 3 mm to 20 mm, in particular 5-18 mm, more particularly 10-16 mm.

6. Absorbent hygiene article according to one of the preceding claims, **characterized in that** the elastic flat material (6) has a longitudinal extent of 90 mm to 250 mm, in particular 100 mm to 240 mm, more particularly 110 mm to 230 mm.

7. Absorbent hygiene article according to one of the preceding claims, **characterized in that** the elastic flat material (6) is in each case arranged completely in the overlap region (5).

8. Absorbent hygiene article according to one of the preceding claims, **characterized in that** a longitudinal extent (20) of the elastic flat material (6) is at least 20%, in particular 20-80%, more particularly 30-70%, more particularly 40-60%, of a longitudinal extent (21) of the hygiene article (1).

9. Absorbent hygiene article according to one of the preceding claims, **characterized in that** the hygiene article (1) has, along the longitudinal direction (11), a front region (12), a rear region (13), and a crotch region (14) arranged between the front region (12) and the rear region (13) and coming to lie between the legs of a user, wherein the elastic flat material (6) in the longitudinal direction (11) is arranged at least in the crotch region (14).

10. Absorbent hygiene article according to one of the preceding claims, **characterized in that** the backsheet (3) has a basis weight of 15-35 g/m$^2$, in particular 17-30, more particularly 19-28 g/m$^2$.

11. Absorbent hygiene article according to one of the preceding claims, **characterized in that** the backsheet (3) consists of a water-impermeable film or a water-impermeable nonwoven film composite material.

12. Absorbent hygiene article according to one of the preceding claims, **characterized in that** the backsheet (3) is substantially white.

13. Absorbent hygiene article according to one of the preceding claims, **characterized in that** the absorption body (4) is in the shape of an hourglass.

14. Absorbent hygiene article according to one of the preceding claims, **characterized in that** the hygiene article (1) is a folded hygiene article (1a) which is folded onto itself, on at least one fold line (8) running in the transverse direction (10), in the direction of the side (16) facing towards the body in such a way that the topsheet (2) comes to lie at least partially on itself and that the backsheet (3) forms a first surface (22) and a second surface (23), which is to be located opposite the first surface (22), of the folded hygiene article (1a), wherein the elastic flat material (6) extends over the at least one fold line (8) in such a way that the elastic flat material (6) is visually perceptible when viewed from the first surface (22) and from the second surface (23) of the folded hygiene article (1a).

15. Absorbent hygiene article according to one of the preceding claims, **characterized in that** the topsheet-side flat material side (6a) has a topsheet-side flat material colour F4, wherein the topsheet (2) in the hygiene article (1) has a topsheet side (2a) facing

14

towards the body and a topsheet side (2b) facing away from the body, wherein the topsheet (2) has, on the topsheet side (2a) facing towards the body, an outer topsheet colour F5, wherein the topsheet-side flat material colour F4 is different from the outer topsheet colour F5, wherein the topsheet (2) has an opacity of at most 90.0%, in particular at most 80.0%, more particularly at most 70.0%, more particularly at most 60.0%, more particularly at most 50.0%, more particularly at most 40.0%, wherein the topsheet-side flat material colour F4 of the elastic flat material (6) within the elastified zones (7) is visually perceptible when viewed from the side (16) of the hygiene article (1) facing towards the body, wherein the respective elastified zones (7) have a further colour F6 when viewed from the side (16) of the hygiene article (1) facing towards the body, wherein a colour distance $\Delta E'$ between (i) the further colour F6 and (ii) the topsheet-side flat material colour F4 of the elastic flat material (6) has a value of at most 50.0.

16. Absorbent hygiene article according to Claim 15, **characterized in that** $\Delta E$ is greater than $\Delta E'$, wherein in particular a ratio of $\Delta E$ to $\Delta E'$ has a value of 1.1-3.5, in particular 1.2-3.0, more particularly 1.3-2.5, more particularly 1.4-2.2.

17. Absorbent hygiene article according to one of the preceding claims, **characterized in that** the topsheet (2) is at least partially permeable to liquid and comprises or consists of a nonwoven material or a film material or a combination thereof.

18. Absorbent hygiene article according to one of the preceding claims, **characterized in that** the topsheet (2) has a basis weight of 5-30 g/m$^2$, in particular 10-28 g/m$^2$, more particularly 15-26 g/m$^2$.

19. Absorbent hygiene article according to one of the preceding claims, **characterized in that** the topsheet (2) is substantially white.

**Revendications**

1. Article hygiénique absorbant (1) comprenant une feuille supérieure (2) agencée sur un côté (16) de l'article hygiénique (1) tourné vers le corps, une feuille arrière (3) agencée sur un côté (17) de l'article hygiénique (1) opposé au corps et un corps absorbant (4) agencé entre la feuille supérieure (2) et la feuille arrière (3), l'article hygiénique (1) présentant une direction longitudinale (11) et une direction transversale (10), la feuille supérieure (2) et la feuille arrière (3) s'étendant chacune dans la direction transversale (10) et dans la direction longitudinale (11) au-delà du corps absorbant (4), et formant ensemble, à l'extérieur du corps absorbant (4), une zone de chevauchement (5), l'article hygiénique (1) étant une protection pour incontinence, une serviette hygiénique ou une serviette hygiénique pour femmes,

l'article hygiénique (1) comprenant, dans la direction transversale (10), de part et d'autre du corps absorbant (4), un matériau plat élastique (6) s'étendant dans la direction longitudinale (11) et agencé entre la feuille supérieure (2) et la feuille arrière (3) et au moins en partie dans la zone de chevauchement (5), le matériau plat élastique (6) formant, conjointement avec la feuille supérieure (2) et la feuille arrière (3), une zone élastifiée (7) dans la zone de chevauchement (5),
le matériau plat élastique (6) présentant, dans l'article hygiénique (1), un côté matériau plat (6a) tourné, côté feuille supérieure, vers la feuille supérieure (2) et, dans l'article hygiénique (1), un côté matériau plat (6b) tourné, côté feuille arrière, vers la feuille arrière (3), le matériau plat (6b) côté feuille arrière présentant une couleur F1 de matériau plat côté feuille arrière, la feuille arrière (3) dans l'article hygiénique (1) présentant un côté (3a) de feuille arrière tourné vers le corps et un côté (3b) feuille arrière opposé au corps,
la feuille arrière (3) présentant, sur le côté (3b) de feuille arrière opposé au corps, une couleur F2 de feuille arrière extérieure, la couleur F1 de matériau plat côté feuille arrière étant différente de la couleur F2 de feuille arrière extérieure,
la feuille arrière (3) présentant une opacité d'au plus de 90,0%, la couleur F1 de matériau plat côté feuille arrière du matériau plat élastique (6) étant visible à l'œil nu à l'intérieur des zones élastifiées (7) depuis le côté opposé au corps (17) de l'article hygiénique (1), une différence de couleur $\Delta E$ entre (i) une couleur F3 de la zone élastifiée respective (7) vue depuis le côté opposé au corps (17) de l'article hygiénique (1) et (ii) la couleur F1 de matériau plat côté feuille arrière du matériau plat élastique (6) ayant une valeur d'au plus 70,0.

2. Article hygiénique absorbant selon la revendication 1, **caractérisé en ce que** la feuille arrière (3) présente une opacité d'au plus 85,0%, en particulier d'au plus 80,0%, et, plus particulièrement encore, d'au plus 75,0%.

3. Article hygiénique absorbant selon l'une des revendications précédentes, **caractérisé en ce que** le matériau plat élastique (6) est un matériau en feuille élastique (9) ou un matériau en mousse élastique.

4. Article hygiénique absorbant selon la revendication 3, **caractérisé en ce que** le matériau en feuille élastique présente une épaisseur allant de 10 à 50μm, en particulier allant de 15 à 45μm, et, plus particulièrement encore, allant de 20 à 40μm.

5. Article hygiénique absorbant selon l'une des revendications précédentes, **caractérisé en ce que** le matériau plat élastique (6) présente une extension transversale allant de 3mm à 20mm, en particulier allant de 5mm à 18mm, plus particulièrement encore, allant de 10mm à 16mm.

6. Article hygiénique absorbant selon l'une des revendications précédentes, **caractérisé en ce que** le matériau plat élastique (6) présente une extension longitudinale allant de 90mm à 250mm, en particulier allant de 100mm à 240mm, plus particulièrement encore, allant de 110mm à 230mm.

7. Article hygiénique absorbant selon l'une des revendications précédentes, **caractérisé en ce que** le matériau plat élastique (6) est agencé dans sa totalité dans la zone de chevauchement (5).

8. Article hygiénique absorbant selon l'une des revendications précédentes, **caractérisé en ce qu'**une extension longitudinale (20) du matériau plat élastique (6) est d'au moins 20%, en particulier de 20 à 80%, plus particulièrement encore, de 30 à 70%, plus particulièrement encore, de 40 à 60%, d'une extension longitudinale (21) de l'article hygiénique (1).

9. Article hygiénique absorbant selon l'une des revendications précédentes, **caractérisé en ce que** l'article hygiénique (1) présente, le long de la direction longitudinale (11), une zone avant (12), une zone arrière (13) et une zone d'entrejambe (14) agencée entre la zone avant (12) et la zone arrière (13), destinée à se trouver entre les jambes d'un utilisateur, le matériau plat élastique (6) étant agencé dans la direction longitudinale (11) au moins dans la zone d'entrejambe (14).

10. Article hygiénique absorbant selon l'une des revendications précédentes, **caractérisé en ce que** la feuille arrière (3) présente un grammage allant de 15 à 35g/m$^2$, en particulier allant de 17 à 30g/m$^2$, et plus particulièrement encore, allant de 19 à 28g/m$^2$.

11. Article hygiénique absorbant selon l'une des revendications précédentes, **caractérisé en ce que** la feuille arrière (3) est constituée d'un film imperméable à l'eau ou d'un matériau composite imperméable à l'eau composé d'un film et d'un non-tissé.

12. Article hygiénique absorbant selon l'une des revendications précédentes, **caractérisé en ce que** la feuille arrière (3) est sensiblement blanche.

13. Article hygiénique absorbant selon l'une des revendications précédentes, **caractérisé en ce que** le corps absorbant (4) est en forme de sablier.

14. Article hygiénique absorbant selon l'une des revendications précédentes, **caractérisé en ce que** l'article hygiénique (1) est un article hygiénique plié (1a) qui est plié sur lui-même au moins au niveau d'une ligne de pliage (8) s'étendant dans la direction transversale (10) en direction du côté (16) tourné vers le corps, de telle sorte que la feuille supérieure (2) vienne se superposer au moins par endroits sur elle-même et que la feuille arrière (3) forme une première surface (22) et une deuxième surface (23) de l'article hygiénique plié (1a), qui est située à l'opposé de la première surface (22), le matériau plat élastique (6) s'étendant au-delà de ladite au moins une ligne de pliage (8) de telle sorte que le matériau plat élastique (6) soit visible depuis la première surface (22) et depuis la deuxième surface (23) de l'article hygiénique plié (1a).

15. Article hygiénique absorbant selon l'une des revendications précédentes, **caractérisé en ce que** le côté (6a) du matériau plat côté feuille supérieure présente une couleur F4 de matériau plat côté feuille supérieure, la feuille supérieure (2) dans l'article hygiénique (1) présentant un côté (2a) de feuille supérieure tourné vers le corps et un côté (2b) de feuille supérieure opposé au corps, la feuille supérieure (2) présentant sur le côté (2a) de feuille supérieure tourné vers le corps une couleur F5 de la feuille supérieure extérieure, la couleur F4 du matériau plat côté feuille supérieure étant différente de la couleur F5 de la feuille supérieure extérieure, la feuille supérieure (2) présentant une opacité d'au plus 90,0%, en particulier d'au plus 80,0%, plus particulièrement encore, d'au plus 70,0%, plus particulièrement encore, d'au plus 60,0%, plus particulièrement encore, d'au plus 50,0%, plus particulièrement encore, d'au plus 40,0%, la couleur F4 de matériau plat côté feuille supérieure du matériau plat élastique (6) étant visible à l'œil nu à l'intérieur des zones élastifiées (7) depuis le côté (16) de l'article hygiénique (1) tourné vers le corps, les zones élastifiées respectives (7) présentant une autre couleur F6 vue depuis le côté (16) de l'article hygiénique (1) tourné vers le corps, une différence de couleur ΔE' entre (i) ladite autre couleur F6 et (ii) la couleur F4 de matériau plat côté feuille supérieure du matériau plat élastique (6) ayant une valeur d'au plus 50,0.

16. Article hygiénique absorbant selon la revendication 15, **caractérisé en ce que** ΔE est supérieur à ΔE', le rapport ΔE/ΔE' ayant notamment une valeur comprise entre 1,1 et 3,5, en particulier entre 1,2 et 3,0, plus particulièrement encore, entre 1,3 et 2,5, et, plus particulièrement encore, entre 1,4 et 2,2.

17. Article hygiénique absorbant selon l'une des revendications précédentes, **caractérisé en ce que** la feuille supérieure (2) est perméable aux liquides au moins par endroits et comprend ou est constituée d'un matériau non tissé ou d'un matériau en feuille ou d'une combinaison de ceux-ci.

18. Article hygiénique absorbant selon l'une des revendications précédentes, **caractérisé en ce que** la feuille supérieure (2) présente un grammage allant de 5 à 30g/m$^2$, en particulier allant de 10 à 28g/m$^2$, plus particulièrement encore, allant de 15 à 26g/m$^2$.

19. Article hygiénique absorbant selon l'une des revendications précédentes, **caractérisé en ce que** la feuille supérieure (2) est sensiblement blanche.

Fig. 1

Fig. 2

Fig. 3a

Fig. 3b

Fig. 4

EP 4 389 092 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3716927 A1
- EP 2246017 A1
- EP 2848236 A1
- US 2020352796 A